(19) Europäisches Patentamt
European Patent Office
Office européen des brevets

(11) Veröffentlichungsnummer: **0 176 822**
**B1**

(12) # EUROPÄISCHE PATENTSCHRIFT

(45) Veröffentlichungstag der Patentschrift: **07.06.89**

(51) Int. Cl.⁴: **C 07 C 41/34**, C 07 C 41/42, C 07 C 43/04

(21) Anmeldenummer: **85111438.9**

(22) Anmeldetag: **10.09.85**

(54) **Verfahren zur Herstellung von tertiärem Amylmethyläther(TAME).**

(30) Priorität: **29.09.84 DE 3435936**

(43) Veröffentlichungstag der Anmeldung: **09.04.86 Patentblatt 86/15**

(45) Bekanntmachung des Hinweises auf die Patenterteilung: **07.06.89 Patentblatt 89/23**

(84) Benannte Vertragsstaaten: **AT BE DE FR GB IT NL**

(56) Entgegenhaltungen:
**EP-A- 0 082 447**
**AT-B- 348 497**
**DE-A- 3 006 104**
**GB-A- 2 134 905**
**SOVIET INVENTIONS ILLUSTRATED, Section CH, Week B 49, 23 Jänner 1980. DERWENT PUBLICATIONS LTD, London; H 06**

(73) Patentinhaber: **Linde Aktiengesellschaft**
**Abraham-Lincoln-Strasse 21**
**D-6200 Wiesbaden (DE)**

(72) Erfinder: **Wernicke, Hans-Jürgen, Dr. rer. nat.**
**Böhmerwaldstrasse 37**
**D-8192 Geretsried (DE)**
Erfinder: **Glatthaar, Reinhard, Dipl.-Ing.**
**Heiterwanger Strasse 34**
**D-8000 München 70 (DE)**
Erfinder: **Buckl, Nikolaus, Dr. rer. nat.**
**Aubinger Strasse 117**
**D-8000 München 60 (DE)**

(74) Vertreter: **Schaefer, Gerhard, Dr.**
**Linde Aktiengesellschaft Zentrale Patentabteilung**
**D-8023 Höllriegelskreuth (DE)**

**EP 0 176 822 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft ein Verfahren zur Herstellung von tertiärem Amylmethyläther (TAME), bei dem ein Einsatzstrom, der im wesentlichen $C_5$-Kohlenwasserstoffe enthält oder aus einer Leichtbenzinfraktion aus einer thermischen oder katalytischen Spaltung besteht, unter Zufügung von Methanol veräthert wird, das bei der Verätherung gebildete Produkt durch Destillation in eine Kopffraktion, die im wesentlichen nichtreagierte leichte Kohlenwasserstoffe und nichtreagiertes Methanol enthält, sowie eine Sumpffraktion die im wesentlichen TAME enthält, zerlegt, die Kopffraktion verflüssigt und aus der verflüssigten Kopffraktion das Methanol abgetrennt wird.

Neben Methyl-t-Butyläther sind auch höhere, tertiäre Äther geeignete Zusätze zu Vergaserkraftstoffen. Der Ätherzusatz dient unter anderem zur Steigerung der Oktanzahl des Kraftstoffes. Die Herstellung der Äther erfolgt durch selektive katalytische Verätherung der entsprechenden Kohlenwasserstofffraktionen mit Alkohol.

Bei einem bekannten Verfahren dieser Art (Chemical Engineering Progress, August 1982, Seiten 36 bis 45) wird ein $C_5$-Kohlenwasserstoff-Einsatzstrom mit Methanol vermischt und einem Reaktor zugeführt. Dort wird mittels eines selektiv wirkenden Katalysators ein Teil des Einsatzes zu tertiärem Amylmethyläther (TAME) umgesetzt. In dem aus dem Reaktor abströmenden Produkt sind neben TAME noch unreagierte Kohlenwasserstoffe und unreagiertes Methanol enthalten. Dieses Gemisch wird zur Abtrennung des Methanols einer Destillationskolonne zugeführt, in der mittels aceotroper Destillation eine Zerlegung in eine das Methanol sowie einen Teil der unreagierten $C_5$-Kohlenwasserstoffe enthaltendes Kopfprodukt und ein den TAME sowie nichtreagiertes $C_5$-Kohlenwasserstoffe enthaltendes Sumpfprodukt erfolgt. Die $C_5$-Kohlenwasserstoffe werden mittels einer Wasserwäsche von dem Methanol abgetrennt. Im Anschluß daran wird das Wasser in einer Destillationskolonne von dem Methanol abgetrennt.

Aus der GB-A-2 134 905 ist weiterhin ein Verfahren bekannt, welches lediglich die Herstellung von Methyl-t-Butyläther (MTBE) vorsieht. Dabei wird das Verätherungsprodukt mittels Fraktionierung in eine methanol- und kohlenwasserstoffreiche Kopffraktion und eine MTBE-Sumpf-Fraktion zerlegt und die Kopffraktion anchließend gekühlt und dabei kondensiert, wobei sich zwei flüssige Phasen ausbilden, die getrennt weiterverarbeitet werden. Die kohlenwasserstoffreiche Fraktion wird zur Abtrennung restlichen Methanols einer Wasserwäsche unterzogen, wodurch auch eine anschließende Methanol/Wasser-Destillation notwendig ist.

Diese Verfahren besitzen jedoch den Nachteil, daß die Abtrennung des Methanols relativ aufwendig ist, da eine Wasserswäsche und eine Methanol/Wasser-Destillation durchgeführt werden muß.

Zudem ist der GB-A-2 134 905 nirgendwo ein Hinweis zu entnehmen, daß das Verfahren sich auch auf die Herstellung anderer Äther, insbesondere TAME, anwenden läßt.

Der voliegenden Erfindung liegt daher die Aufgabe zugrunde, ein Verfahren der eingangs genannten Art zu entwickeln, das mit geringem Aufwand eine kontinuierliche Rückgewinnung von überschüssigem Methanol ermöglicht.

Diese Aufgabe wird erfindungsgemäß dadurch gelöst, daß die verflüssigte Kopffraktion bis unterhalb der kritischen Entmischungstemperatur abgekühlt und in zwei Phasen getrennt wird, von denen die eine im wesentlichen das Methanol und die andere im wesentlichen das Methanol und die andere im wesentlichen die Kohlenwasserstoffe enthält, und die beiden Phasen getrennt voneinander abgezogen werden.

Es wurde in Rahmen der Erfindung festgestellt, daß sich das Flüssigkeitsgemisch, das sich bei der Verflüssigung der Kopffraktion bildet, unter Abkühlung schnell entmischt, wobei eine den größten Teil des Methanols enthaltende Phase gewonnen wird. Bei der Entmischung bildet sich eine schwerere untere Schicht, die im wesentlichen unreagiertes Methanol enthält und eine auf ihr schwimmende obere Schicht, die im wesentlichen die unreagierten Kohlenwasserstoffe enthält. Die beiden Flüssigphasen werden getrennt voneinander aus einem entsprechend gestalteten Behälter entnommen.

Mit dem erfindungsgemäßen Verfahren ist damit auf einfache Weise eine Rückgewinnung des Methanols ermöglicht. Die Trennung der beiden Phasen erfolgt dabei kontinuierlich.

Bei einer bevorzugten Ausgestaltung der erfindungsgemäßen Verfahrens wird die das Methanol enthaltende Phase mindestens teilweise dem Einsatzstrom zur Verätherung zugeführt.

Es hat sich als zweckmäßig erwiesen, wenn die Kopffraktion der Destillation durch Ausnutzung von Kälte aus dem Gaszerlegungsteil einer Analge zur thermischen oder katalytischen Spaltung von Kohlenwasserstoffen oder einer Tieftemperatur-Gaszerlegung abgekühlt wird. Die Kopffraktion wird dabei vor der Trennung der beiden Phasen vorteilhaft auf eine Temperatur von — 60 °C bis + 50 °C abgekühlt.

Gemäß einer bevorzugten Weiterbildung des erfindungsgemäßen Verfahrens wird der Kopffraktion vor der Phasentrennung eine geringfügige Menge Wasser zugemischt. Insbesondere beträgt der zugemischte Wasseranteil zwischen 0,01 und 2 Gew.% der Kopffraktion der Destillation.

Die Zuspeisung von Wasser bewirkt eine bessere Auftrennung des Methanols und der Kohlenwasserstoffe bzw. gestattet eine Phasentrennung bei höherer Temperatur. Das Wasser wird zum überwiegenden Teil zusammen mit der Methanolphase entnommen. Der Wasseranteil ist auch dann unproblematisch,

wenn das Methanol zur Verätherung zurückgeführt wird, da das Wasser die Verätherung nur wenig beeinflußt. Es führt lediglich zur Bildung höherer Alkohole, die bei der Destillation mit dem Sumpfprodukt entnommen werden. Dort stören die höheren Alkohole in vielen Anwendungsfällen, wie bei der Erzeugung von Zusätzen zur Vergaserkrafstoff, nicht, da sie ebenfalls die motorischen Eigenschaften von Fahrbenzin verbessern.

Es hat sich als günstig erwiesen, wenn gemäß einer Weiterbildung des erfindungsgemäßen Verfahrens die Destillation und die Phasentrennung bei einem Druck von 1 bis 20 bar durchgeführt werden.

In bevorzugter Ausführung des erfindungsgemäßen Verfahrens wird TAME in reiner Form oder im Gemisch mit einem Teil der nichtreagierten Kohlenwasserstoffe aus dem Sumpfprodukt der Destillation abgetrennt. Dabei wird TAME üblicherweise in reiner Form oder zusammen mit den nichtreagierten Kohlenwasserstoffen einem Ottokraftstoff zugemischt.

Es ist von Vorteil, wenn mindestens in Teil der die Kohlenwasserstoffe enthaltenden Phase der Sumpffraktion der Destillation zugeführt wird. Diese Verfahrensweise kommt dann zum Einsatz, wenn die nichtreagierenden Einsatzkohlenwasserstoffe als Mischkomponenten für das Produkt verwendet, sind, beispielsweise wenn TAME mit Vergaserkraftstoff vermischt wird.

Es erweist sich weiterhin als zweckmäßig, wenn mindestens ein Teil der die Kohlenwasserstoffe enhaltenden Phase des Entmischungsbehälters der Destillation als Rücklaufflüssigkeit zugeführt wird. Die rückgeführten Kohlenwasserstoffe begünstigen eine vollständige Abtrennung des Methanols als Kopfprodukt bei der Destillation, so daß im ätherhaltigen Produkt entweder Alkoholfreiheit oder ein tolerierbarer Restgehalt eingestellt werden kann.

Das erfindungsgemäße Verfahren ist außer zur Herstellung von TAME auch bei Verwendung anderer Alkohole, beispielsweise Äthanol für die Herstellung von Äthyläther geeignet.

Überdies ist es von Vorteil, wenn wie weiter vorgeschlagen wird, bei der Verätherung ein für die Bildung von tertiärem Amylmethyläther (TAME) selektiver Katalysator eingesetzt wird. Daneben läßt sich das erfindungsgemäße Verfahren auch für die Erzeugung von $C_4$- und $C_{6+}$-Äther einsetzen, die analog aus dem Olefin und Methanol erzeugt werden.

Bei einer bevorzugten Weiterbildung des erfindungsgemäßen Verfahrens wird die Verätherung bei einer Temperatur von + 40 bis + 120 °C und bei einem Absolutdruch von 1,2 bis 20 bar durchgeführt.

Vorzugsweise wird zumindest ein Teil der bei der Verätherung freiwerdenden Reaktionswärme der Destillation zugeführt und/oder zur Anwärmung eines Teils oder des gesamten Einsatzstromes zur Verätherung verwendet. Hierbei wird die Reaktionswärme zur Energieeinsparung bei der Destillationskolonne genutzt.

Die Erfindung sowie weitere Einzelheiten der Erfindung werden anhand von schematisch dargestellten Ausführungsbeispielen für die Erzeugung von TAME näher erläutert.

Es zeigen :

Figur 1 ein Fließschema eines erfindungsgemäßen Verfahrens

Figuren 2 und 3 Zustandsdiagramme.

Das in Figur 1 dargestellte Verfahren dient zur Erzeugung von TAME. TAME wird beispielsweise als Zusatz zur Erhöhung der Klopffestigkeit bleiarmer bzw. bleifreier Vergaserkraftstoffe werdendet. Ein Kohlenwasserstoff-Einsatzstrom 1, beispielsweise eine $C_5$-Fraktion von Pyrolysebenzin oder ein entsprechender FCC-Schnitt, wird zusammen mit Methanol (Leitung 2) einem Verätherungsreaktor 3 zugeführt. Der Verätherungsreaktor 3 enthält einen für die Bildung von TAME selektiv wirkenden Katalysator, beispielsweise Polystyrolharz in der H-Form, sulfonierte Phenolformaldehydharze oder sulfonierte Kohle.

In dem Verätherungsreaktor 3 wird der Kohlenwasserstoff-Einsatzstrom 1 mit dem Methanol veräthert. Als Hauptreaktion findet dabei die Umsetzung von 2-Methylbuten-(1) ($\gamma$-Amylen) und 2-Methylbuten-(2) ($\beta$-Iso-Amylen) mit Methanol zu TAME statt. Der Reaktionsumsatz beträgt etwa 65 %. Bei geeigneter Wahl des Katalysators reagieren alle anderen Komponenten der $C_5$-Fraktion mit Methanol nicht. In geringem Umfang treten folgende Nebenreaktion auf :

$$\text{Iso-Amylen} + H_2O \leftrightharpoons \text{t-Amylalkohol}$$
$$\text{Methanol} + \text{Methanol} \leftrightharpoons \text{Dimethyläther} + H_2O$$
$$2\ \text{Iso-Amylen} \leftrightharpoons \text{Di-Iso-Amylen}$$

Ein Teil des Kohlenwasserstoffeinsatzes und des Methanols durchströmt den Verätherungsreaktor 3 ohne zu reagieren. Demgemäß wird aus dem Verätherungsreaktor 3 ein Produktstrom 4 abgezogen, der TAME sowie die obengenannten Nebenprodukte und unreagierte Komponenten des Einsatzes enthält. Der Produktstrom 4 wird einer Destillationskolonne 5 zugeführt und bei einem Druck von beispielsweise 1,6 bar in eine Kopf- und eine Sumpffraktion zerlegt. Der Trennschnitt in der Destillationskolonne 5 ist so gelegt, daß über Kopf ein Gemisch von unreagiertem Methanol und unreagierten Kohlenwasserstoffen (im wesentlichen $C_5$-Kohlenwasserstoffen) abgezogen wird (Leitung 6), während die Sumpffraktion, die über eine Leitung 7 entnommen wird, hauptsächlich TAME enthält. Daneben sind in der Sumpffraktion noch höhere Alkohole und Oligomere enthalten, die als Nebenprodukt bei der Verätherung gebildet wurden.

Die Kopffraktion wird in einem Kühler 8 auf eine Temperatur unterhalb der kritischen Entmischungstemperatur abgekühlt. Dabei wird die Kopffraktion verflüssigt. Das Flüssigkeitsgemisch wird einem Behälter 9 zugeführt, in welchem sich das Flüssigkeitsgemisch in zwei flüssige Phasen von unterschiedlicher Dichte entmischt. Eine schwerere methanolreiche Phase, die noch 10 bis 50 %, vorzugsweise 25 bis 40 % Kohlenwasserstoffe enthält, sammelt sich am Boden des Behälters 9, auf ihr schwimmt eine kohlenwasserstoffreiche Phase. Der Behälter 9 ist als Dekanter ausgebildet und ermöglicht die getrennte Entnahme der beiden Phasen.

Die methanolreiche Phase wird über eine Leitung 10 entnommen und dem Methanol-Einsatzstrom 2 zugemischt. Die kohlenwasserstoffreiche Phase wird über eine Leitung 11 entnommen und, wenn ein Methanol- und Kohlenwasserstoff-Restgehalt im TAME zulässig ist, dem TAME-Kohlenwasserstoffgemisch in Leitung 7 über die gestrichelt dargestellte Leitung 15 zugemischt (Leitung 12). Dies ist möglich, wenn der TAME zur Veredelung von Vergaserkraftstoff verwendet wird. Zweckmäßigerweise münden die beiden Entnahmeleitungen 10 und 11 übereinander in die Phasentrenneinrichtung.

Ein Teil der kohlenwasserstoffreichen Fraktion 11 kann bei einer anderen Verfahrensvariante über eine ebenfalls gestrichelt dargestellte Leitung 13 auf die Destillationskolonne 5 aufgegeben werden, um den Methanolgehalt in einem aus dem Sumpf abzuziehenden TAME-Kohlenwasserstoff-Gemisch zu reduzieren. In einer weiteren Verfahrensvariante (nicht dargestellt) kann der Methanolgehalt in der kohlenwasserstoffreichen Phase (Leitung 11) durch eine einfache Abtriebsäule vermindert werden.

Bei einer ebenfalls möglichen Verfahrensvariante wird über eine gestrichelt dargestellte Leitung 14 eine geringfügige Menge Wasser in das Kopfprodukt aus der Destillationskolonne 5 eingeleitet. Das Wasser bewirkt eine bessere Entmischung der beiden Phasen bzw. erlaubt die Trennung bei höherer Temperatur in dem Behälter 9. Das Wasser gelangt praktisch vollständig in das über Leitung 10 zurückgeführte Methanol. Es beeinflußt die Verätherungsreaktionen nur geringfügig und führt zur Bildung höherer Alkohole, die mit dem Sumpfprodukt aus der Destillationskolonne entnommen werden.

Im folgenden sind Zahlenbeispiele für das erfindungsgemäße Verfahren angegeben, die den Prozeßstufen gemäß Fig. 1 entsprechen :

Kohlenwasserstoff-Einsatzstrom (1) : Leichtbenzin-Fraktion eines Steam-Crackers mit folgender Zusammensetzung : Gehalt an reaktiven Kohlenwasserstoffen : 7,8 Gew.% 2M-Buten-1, 17,6 Gew.% 2M-Buten-2.

Verätherung : Temperatur 70 °C, Druck 15 bar
Molverhältnis Methanol/reaktive Kohlenwasserstoffe 1,1/1
Gesamtumsatz : ca. 65 %
Selektivität : über 90 %

Reaktionsprodukt (4) : 19,2 Gew.% TAME, 5,4 Gew.% Methanol, 75,1 Gew.% Kohlenwasserstoffe, 0,3 Gew.% sonstige Produkte. Das Reaktionsprodukt (4) wird nun zwei verschiedenen Fraktionierungsverfahren unterworfen,

Beispiel 1

Destillation (5) :

| Gew.% | Kopfprodukt | Sumpfprodukt |
|---|---|---|
| TAME | 0,1 | 93,6 |
| Methanol | 6,7 | < 0,1 |
| Kohlenwasserstoffe | 93,2 } | |
| sonstige Produkte | - } | 6,3 |

Phasentrennung (9) :
Temperatur — 10 °C, Druck 1,6 bar, 0,3 Gew.% $H_2O$ bezogen auf das Kopfprodukt (6).

| Gew.% | Methanol-Phase*) | Kohlenwasserstoffphase |
|---|---|---|
| TAME | 0,1 | 0,1 |
| Methanol | 56,1 | 3,7 |
| Wasser | 3,6 | < 0,1 |

*) Rückführung zum Verätherungsreaktor 3, erforderlichenfalls nach Trocknung

| | |
|---|---|
| Verätherungsprodukt (7) : | |
| TAME | 93,6 Gew.% |
| Methanol | < 0,1 Gew.% |
| Kohlenwasserstoffe und sonstige Prod. | 6,3 Gew.% |

| | |
|---|---|
| ROZ (Research Oktanzahl), unverbleit | 109 |
| MOZ (Motor Oktanzahl), unverbleit | 98 |

## Beispiel 2

Azeotropdestillation (5) :

| Gew.% | Kopfprodukt | Sumpfprodukt |
|---|---|---|
| TAME | 0,9 | 33,6 |
| Methanol | 11,8 | 0,3 |
| Kohlenwasserstoffe | 87,3 } | 66,1 |
| sonstige Produkte | - } | |

Phasentrennung (9) :
Temperatur — 10 °C, Druck 1,6 bar, 0,3 Gew.% $H_2O$ bezogen auf das Kopfprodukt (6).

| Gew.% | Methanolphase *) | Kohlenwasserstoffphase |
|---|---|---|
| TAME | 0,9 | 0,9 |
| Methanol | 57,7 | 4,5 |
| Wasser | 1,5 | < 0,1 |

*) Rückführung zum Verätherungsreaktor 3, erforderlichenfalls nach Trocknung

Gegenüber Beispiel 1 ist die Kohlenwasserstoffmenge deutlich verringert.

Verätherungsprodukt (12) (entspricht Mischung aus Verätherungsprodukt (7) und kohlenwasserstoffreicher Phase (11)

| | |
|---|---|
| TAME | 20,6 Gew.% |
| Methanol | 1,7 Gew.% |
| Kohlenwasserstoffe und sonstige Produkte | 77,7 Gew.% |

| | |
|---|---|
| ROZ (Research Oktanzahl), unverbleit | 102 |
| MOZ (Motor Oktanzahl), unverbleit | 89 |

Die Figuren 2 und 3 zeigen die Grenzlinie des Zweiphasengebietes für Mischungen aus Methanol, dem Kohlenwasserstoffeinzatz (gemäß Zahlenbeispiel) und Wasser. Aufgetragen ist die Temperatur über der Methanolkonzentration des dem Behälter 9 zugeführten Stromes 6 bei Drücken von 1 bzw. 5 bar und bei folgenden unterschiedlichen Wassergehalten :

| Kurve Nr. | Wassergehalt im Gemisch- Strom aus (6) und (14) Gew.% |
|---|---|
| 14 | 0 |
| 15 | 0,15 |
| 16 | 0,30 |
| 17 | 0,45 |
| 18 | 0,60 |

**Patentansprüche**

1. Verfahren zur Herstellung von tertiärem Amylmethyläther (TAME), bei dem ein Einsatzstrom, der im wesentlichen $C_5$-Kohlenwasserstoffe enthält oder aus einer Leichtbenzinfraktion aus einer thermischen oder katalytischen Spaltung besteht, under Zufügung von Methanol veräthert wird, das bei der Verätherung gebildete Produkt durch Destillation in eine Kopffraktion, die im wesentlichen nichtreagierte leichte Kohlenwasserstoffe und nichtreagiertes Methanol enthält, sowie eine Sumpffraktion, die im wesentlichen TAME enthält, zerlegt, die Kopffraktion verflüssigt und aus der verflüssigten Kopffraktion das Methanol abgetrennt wird, dadurch gekennzeichnet, daß die verflüssigte Kopffraktion bis unterhalb der kritischen Entmischungstemperatur abgekühlt und in zwei Phasen getrennt wird, von denen die eine im wesentlichen das Methanol und die andere in wesentlichen die Kohlenwasserstoffe enthält, und die beiden Phasen getrennt voneinander abgezogen werden.

2. Verfahren nach Anspruch 1, dadurch gekennzeichnet, daß die das Methanol enthaltende Phase mindestens teilweise dem Einsatzstrom zur Verätherung zugeführt wird.

3. Verfahren nach Anspruch 1 oder 2, dadurch gekennzeichnet, daß die Kopffraktion der Destillation durch Ausnutzung von Kälte aus dem Gaszerlegungsteil einer Anlage zur thermischen oder katalytischen Spaltung von Kohlenwasserstoffen oder einer Tieftemperatur-Gaszerlegung abgekühlt wird.

4. Verfahren nach einem der Ansprüche 1 bis 3, dadurch gekennzeichnet, daß die Kopffraktion vor der Trennung der beiden Phasen auf eine Temperatur von — 60 bis + 50 °C abgekühlt wird.

5. Verfahren nach einem der Ansprüche 1 bis 4, dadurch gekennzeichnet, daß der Kopffraktion vor der Phasentrennung eine geringfügige Menge Wasser zugemischt wird.

6. Verfahren nach Anspruch 5, dadurch gekennzeichnet, daß der zugemischte Wasseranteil zwischen 0,01 und 2 Gew.% der Kopffraktion der Destillation beträgt.

7. Verfahren nach einem der Ansprüche 1 bis 6, dadurch gekennzeichnet, daß die Destillation und die Phasentrennung bei einem Druck von 1 bis 20 bar durchgeführt werden.

8. Verfahren nach einem der Ansprüche 1 bis 7, dadurch gekennzeichnet, daß TAME in reiner Form oder im Gemisch mit einem Teil der nichtreagierten Kohlenwasserstoffe aus dem Sumpfprodukt der Destillation abgetrennt wird.

9. Verfahren nach einem der Ansprüche 1 bis 8, dadurch gekennzeichnet, daß mindestens ein Teil der die Kohlenwasserstoffe enthaltenden Phase der Sumpffraktion zugeführt wird.

10. Verfahren nach einem der Ansprüche 1 bis 9, dadurch gekennzeichnet, daß mindestens ein Teil der die Kohlenwasserstoffe enthaltenden Phase der Destillation als Rücklaufflüssigkeit zugeführt wird.

11. Verfahren nach einem der Ansprüche 1 bis 10, dadurch gekennzeichnet, daß bei der Verätherung ein für die Bildung von tertiärem Amylmethyläther (TAME) selektiver Katalysator eingesetzt wird.

12. Verfahren nach einem der Ansprüche 1 bis 11, dadurch gekennzeichnet, daß die Verätherung bei einer Temperatur von + 40 bis + 120 °C und bei einem Absolutdruck von 1,2 bis 20 bar durchgeführt wird.

13. Verfahren nach einem der Ansprüche 1 bis 12, dadurch gekennzeichnet, daß zumindest ein Teil der bei der Verätherung freiwerdenden Reaktionswärme der Destillation zugeführt und/oder zur Anwärmung eines Teils oder des gesamten Einsatzstromes zur Verätherung verwendet wird.

**Claims**

1. A process for the production of tertiary amylmethyl ether (TAME) in which an input stream which essentially contains $C_5$-hydrocarbons or consists of a light gasoline fraction from thermal or catalytic cracking, is etherified by the addition of methanol, the product formed by the etherification is separated into a head fraction which essentially contains unreacted, light hydrocarbons and unreacted methanol, and a sump fraction which essentially contains TAME, the head fraction is liquefied and the methanol is separated from the liquefied head fraction, characterised in that the liquefied head fraction is cooled to below the critical demixing temperature and is separated into two phases, one of which essentially contains the methanol and the other essentially contains the hydrocarbons, and the two phases are withdrawn separately.

2. A process as claimed in Claim 1, characterised in that the phase containing the methanol is fed at least in part to the input stream for the etherification.

3. A process as claimed in Claim 1 or 2, characterised in that the head fraction from the distillation is cooled using cold from the gas separation section of a plant for the thermal or catalytic cracking of hydrocarbons or from a low-temperature gas separation unit.

4. A process as claimed in one of Claims 1 to 3, characterised in that, prior to the separation of the two phases, the head fraction is cooled to a temperature of — 60 to + 50 °C.

5. A process as claimed in one of Claims 1 to 4, characterised in that a small amount of water is mixed with the head fraction prior to the phase separation.

6. A process as claimed in Claim 5, characterised in that the added water amounts to between 0.01 and 2 weight % of the head fraction of the distillation.

7. A process as claimed in one of Claims 1 to 6, characterised in that the distillation and the phase separation are carried out at a pressure of 1 to 20 bar.

8. A process as claimed in one of Claims 1 to 7, characterised in that TAME is separated from the sump product of the distillation in a pure form or mixed with a part of the unreacted hydrocarbons.

9. A process as claimed in one of Claims 1 to 8, characterised in that at least a part of the phase containing the hydrocarbons is fed to the sump fraction.

10. A process as claimed in one of Claims 1 to 9, characterised in that at least a part of the phase containing the hydrocarbons is fed to the distillation step as reflux liquid.

11. A process as claimed in one of Claims 1 to 10, characterised in that, during the etherification, a catalyst is used which is selective for the formation of tertiary amylmethyl ether (TAME).

12. A process as claimed in one of Claims 1 to 11, characterised in that the etherification is carried out at a temperature of + 40 to + 120 °C and at an absolute pressure of 1.2 to 20 bar.

13. A process as claimed in one of Claims 1 to 12, characterised in that at least a part of the heat of reaction released during the etherification is supplied to the distillation step and/or is used to heat a part or the whole of the input stream for the etherification.

**Revendications**

1. Procédé pour la préparation de tertio-amylméthyl-éther (TAME) dans lequel un courant d'alimentation contenant essentiellement des hydrocarbures en $C_5$ ou comportant une fraction d'essences légères résultant d'un craquage thermique ou catalytique, après addition de méthanol, subit une éthérification et dans lequel le produit de cette éthérification est scindé, par distillation, en une fraction de tête qui contient essentiellement des hydrocarbures légers et du méthanol n'ayant pas réagi et en une fraction de cuve qui contient essentiellement du TAME, la fraction de tête est condensée et le méthanol est séparé de cette fraction de tête condensée, ce procédé étant caractérisé en ce que la fraction de tête condensée est refroidie en dessous de la température critique de démixtion du mélange puis séparée en deux phases dont la première est essentiellement formée du méthanol et la seconde des hydrocarbures, et en ce que les deux phases sont séparées.

2. Procédé selon la revendication 1, caractérisé en ce que la phase contenant le méthanol est au moins partiellement recyclée dans le courant d'alimentation du réacteur d'éthérification.

3. Procédé selon la revendication 1 ou 2, caractérisé en ce que la fraction de tête de la distillation est refroidie par utilisation des frigories de la fraction gazeuse provenant d'un séparateur d'une installation de dissociation thermique ou catalytique d'hydrocarbures ou d'une installation de séparation des gaz à basse température.

4. Procédé selon l'une quelconque des revendications 1 à 3, caractérisé en ce que la fraction de tête est refroidie à une température entre — 60 et + 50 °C avant la séparation des deux phases.

5. Procédé selon l'une quelconque des revendications 1 à 4, caractérisé en ce que avant séparation des phases, une petite quantité d'eau est ajoutée à la fraction de tête.

6. Procédé selon la revendication 5, caractérisé en ce que la proportion d'eau mélangée à la fraction de tête est comprise entre 0,01 et 2 % en poids par rapport à la fraction de tête.

7. Procédé selon l'une quelconque des revendications 1 à 6, caractérisé en ce que la distillation et la séparation de phase sont mises en œuvre à une pression comprise entre 1 et 20 bars.

8. Procédé selon l'une quelconque des revendications 1 à 7, caractérisé en ce que l'on recueille comme produit de cuve dans la distillation le TAME pur ou en mélange avec une partie des hydrocarbures n'ayant pas réagi.

9. Procédé selon l'une quelconque des revendications 1 à 8, caractérisé en ce qu'au moins une partie de la phase contenant les hydrocarbures est amenée à la fraction de cuve.

10. Procédé selon l'une quelconque des revendications 1 à 9, caractérisé en ce qu'au moins une partie de la phase contenant des hydrocarbures produit dans la distillation est utilisée en tant que reflux.

11. Procédé selon l'une quelconque des revendications 1 à 10, caractérisé en ce que dans la réaction d'éthérification est utilisé un catalyseur sélectif pour la formation de tertioamylméthyléther (TAME).

12. Procédé selon l'une quelconque des revendications 1 à 11, caractérisé en ce que l'on conduit la réaction d'éthérification à une température comprise entre — 40 et + 120 °C et sous une pression absolue de 1,2 à 20 bars.

13. Procédé selon l'une quelconque des revendications 1 à 12, caractérisé en ce qu'au moins une partie de la chaleur libérée lors de l'éthérification est envoyée à la distillation et/ou sert à chauffer tout ou partie du courant d'alimentation du réacteur d'éthérification.

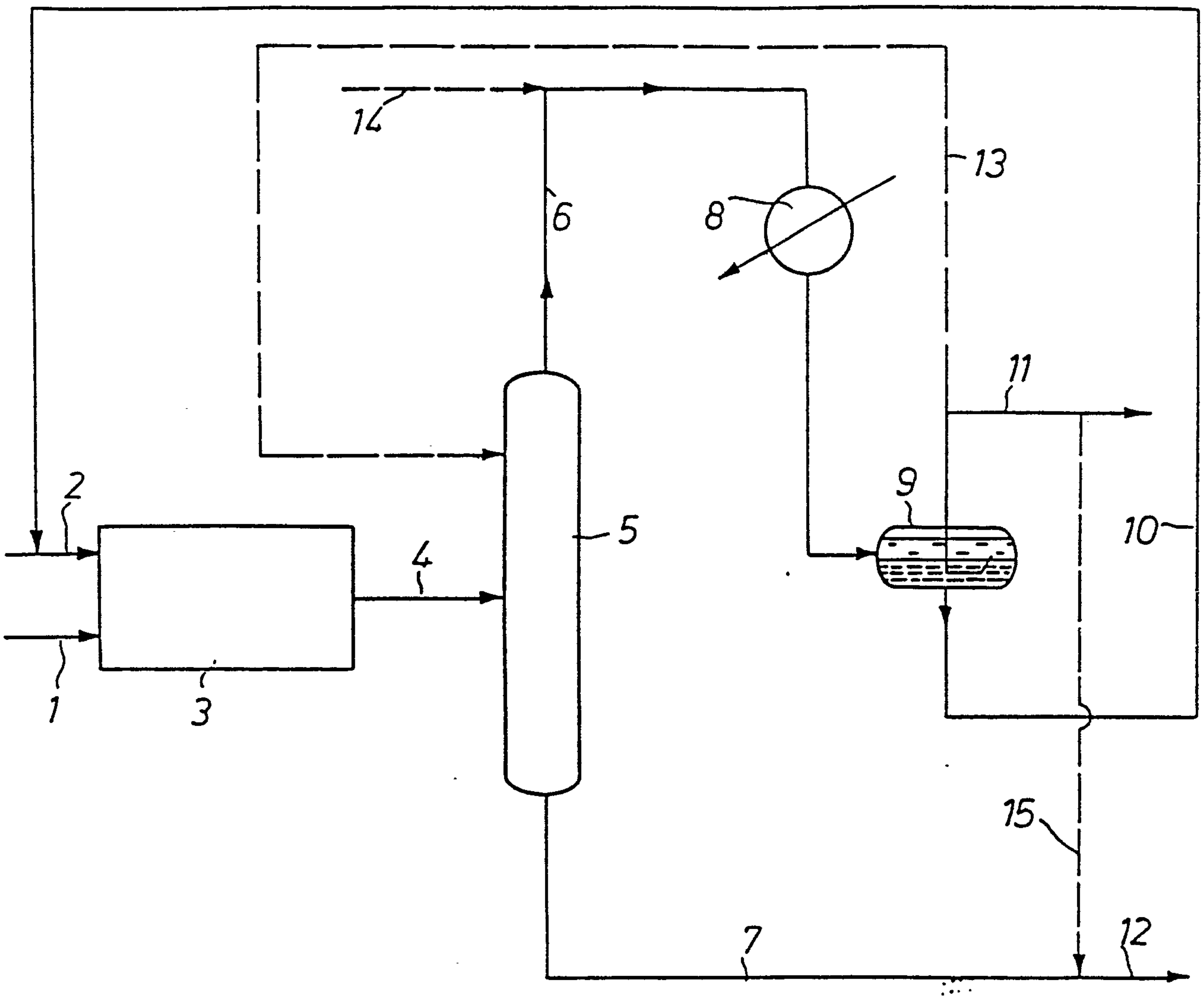


Fig.1

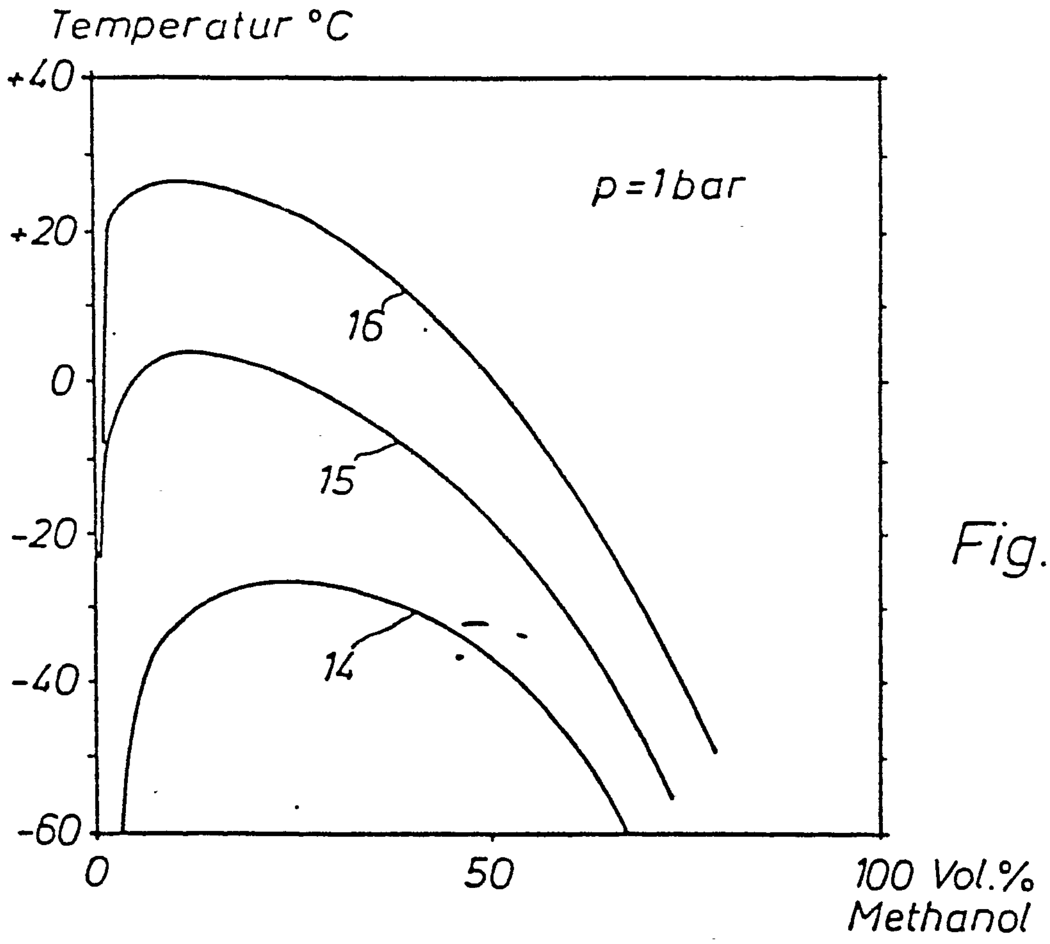
Temperatur °C
+40
+20
0
-20
-40
-60
p = 1 bar
16
15
14
0
50
100 Vol.%
Methanol

Fig. 2

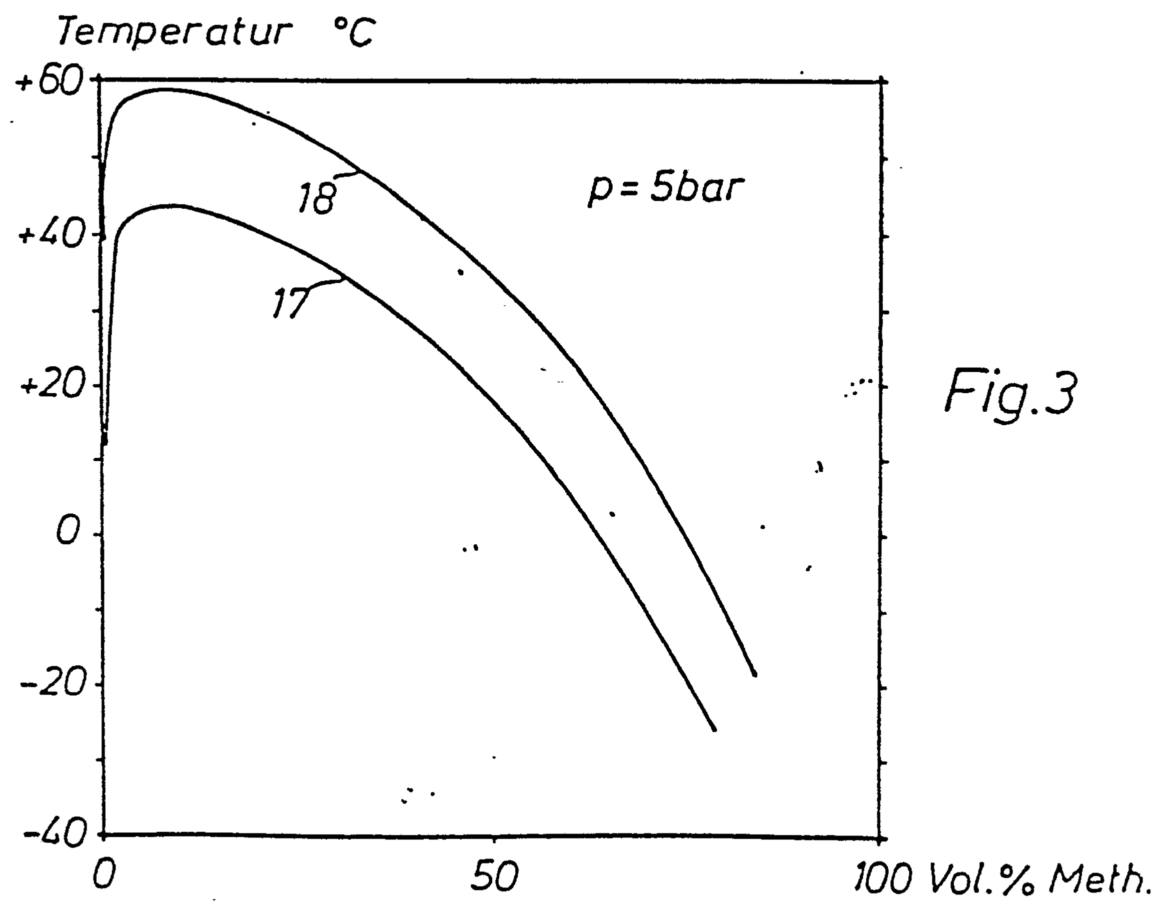
Temperatur °C
+60
+40
+20
0
-20
-40
p = 5 bar
18
17
0
50
100 Vol.% Meth.

Fig.3